# EUROPEAN PATENT APPLICATION

(11) **EP 3 960 176 A1**
(43) Date of publication of application: **02.03.2022**
(21) Application number: 20192982.5
(22) Date of filing: 26.08.2020
(51) Int. Cl.: A61K 31/4706, A61K 31/496, A61K 31/662, A61K 45/06, A61P 11/00, A61P 31/14

(54) **PIPERAQUINE AND RELATED DRUG COMBINATIONS FOR USE IN THE TREATMENT OF COVID-19**

(71) Applicant: DMG Deutsche Malaria GmbH, 20354 Hamburg (DE)
(72) Inventor: HUTCHINSON, David, Westerham, Kent TN16 2DR (GB); LINDEMANN, Björn, 20459 Hamburg (DE)
(74) Representative: Simandi, Claus

(57) **Abstract**

The present invention relates to pharmaceutical preparations / compositions comprising 4-aminoquinoline derivatives, in particular Piperaquine, Chloroquine, or Hydroxychloroquine as active ingredients, and in a further embodiment in combination with 3-N-formyl hydroxy amino propyl phosphonic acid derivatives or 3-N-acetyl hydroxy amino propyl phosphonic acid derivatives as active ingredients and optionally, in combination with an immunosuppressant for use in the prophylaxis and treatment of infections caused by RNA-viruses.

## Description

The present invention relates to pharmaceutical preparations / compositions comprising 4-aminoquinoline derivatives, in particular Piperaquine, Chloroquine, or Hydroxychloroquine as active ingredients, and in a further embodiment in combination with 3-N-formyl hydroxy amino propyl phosphonic acid derivatives or 3-N-acetyl hydroxy amino propyl phosphonic acid derivatives as active ingredients and optionally, in combination with an immunosuppressant for use in the prophylaxis and treatment of infections caused by RNA-viruses.

Coronaviruses (CoV) are a large family of viruses that cause illness ranging from the common cold to more severe diseases such as Middle East Respiratory Syndrome (MERS-CoV) and Severe Acute Respiratory Syndrome (SARS-CoV). A novel coronavirus (nCoV) is a new strain, recently named SARS-CoV-2 by the World Health Organization, that had not been previously identified in humans and causing a specific disease named Covid-19.

Coronaviruses are zoonotic, meaning they are transmitted between animals and humans. Detailed investigations found that SARS-CoV was probably transmitted from civet cats to humans and MERS-CoV from dromedary camels to humans. Several known coronaviruses are circulating in animals that have not yet infected humans. Common signs of infection include respiratory symptoms, fever, cough, shortness of breath and breathing difficulties. In more severe cases, infection can cause pneumonia, severe acute respiratory syndrome, kidney failure and even death or long-term consequence.

There is an urgent need for effective treatments for diseases caused by coronaviruses, especially Covid-19.

SARS-CoV and MERS-CoV are both RNA-viruses causing specific interaction with the infected or non-infected immune system. It appears that such interaction is surprisingly comparable with interaction caused by bacteria or parasites. The mode of action is not well understood but 4-aminoquinoline derivatives may increase endosomal pH and glycosylation of the ACE-2 receptor thereby blocking viral invasion of susceptible cells (Devaux CA, Rolain JM, Colson P, et al. New insights on the antiviral effects of chloroquine against coronavirus: what to expect for COVID-19? https://doi.org/10.1016/j.ijantimicag.2020.105938). The state of the art discusses this issue for Chloroquine or Hydroxychloroquine without considering Piperaquine. However, Piperaquine is well tolerated by human and has less side-effects. Indeed, Chloroquine or Hydroxychloroquine are recently withdrawn from further proceedings of regulatory approval (e.g.: COVID-19: chloroquine and hydroxychloroquine only to be used in clinical trials or emergency use programmes EMA/170590/2020). Recently, it was found that ACE2 proteins are overexpressed in human olfactory epithelium relative to upper airway epithelial cells. This may explain COVID-19-associated olfactory dysfunction (Mengfei Chen, Wenjuan Shen, Nicholas R. Rowan, Heather Kulaga, Alexander Hillel, Murugappan Ramanathan Jr, Andrew P. Lane, levated ACE2 expression in the olfactory neuroepithelium: implications for anosmia and upper respiratory SARS-CoV-2 entry and replication European Respiratory Journal 2020; DOI: 10.1183/13993003.01948-2020).

Hereto, it is assumed that ACE-2 (angiotensin-converting enzyme 2) receptor is the target for mechanism of cellular entry of viruses like SARS-CoV-2 and other RNA-viruses.

Hence, the inventors have found an interaction between 4-aminoquinoline derivatives, preferably Piperaquine, and ACE-2 (angiotensin-converting enzyme 2) receptor, wherein the mode of action can be modulated by 4-aminoquinoline derivatives, preferably Piperaquine, and also in combination with 3-N-formyl hydroxy amino propyl phosphonic acid derivatives or 3-N-acetyl hydroxy amino propyl phosphonic acid derivatives.

Surprisingly, besides 4-aminoquinoline derivatives, in particular Piperaquine, said combination with 3-N-formyl hydroxy amino propyl phosphonic acid derivatives or 3-N-acetyl hydroxy amino propyl phosphonic acid derivatives have a hyper additive effect although those are known as antibiotics.

Hence, the object of the invention refers to pharmaceutical preparations / compositions for use in the prophylaxis and treatment of infections caused by RNA-viruses.

Piperaquine (*PIP,* see below) is a 4-aminoquinoline derivative and well known for the treatment of malaria. Piperaquine is a bisquinoline first synthesized in the 1960s, and used extensively in China and Indochina as prophylaxis and treatment of malaria. Usage declined in the 1980s as piperaquine-resistant strains of P. falciparum arose and artemisinin-based antimalarials became available (e.g. Dihydroartemisinin-piperaquine (Eurartesim)).

Piperaquine (PIP) is 7-chloro-4-[4-[3-[4-(7-chloroquinolin-4-yl)piperazin-1-yl]propyl] piperazin-1-yl]quinoline (CAS No. 4085-31-8) represented by formula (I)

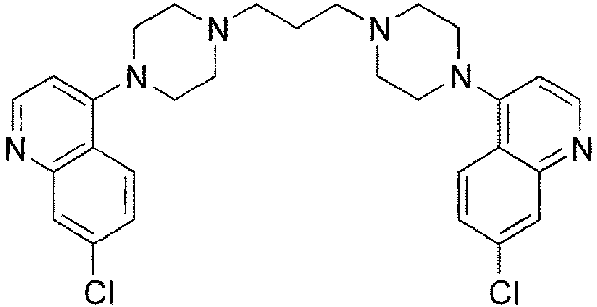

Hence, in a first aspect of the invention, the invention relates to a pharmaceutical composition comprising a 4-aminoquinoline derivative, namely Piperaquine for use in the prophylaxis and treatment of infections caused by an RNA-virus, in particular caused by coronavirus (CoV), in particular caused by Middle East Respiratory Syndrome (MERS)-CoV or Severe Acute Respiratory Syndrome (SARS)-CoV, in particular Acute Respiratory Syndrome (SARS)-CoV-2.

Hereto, in a further aspect of the invention, the invention relates to a pharmaceutical composition comprising a 4-aminoquinoline derivative, namely Piperaquine for use in the prophylaxis and treatment of infections caused by an RNA-virus, wherein the infection leads to a disease, in particular at least one disease selected from the group of respiratory diseases, lower respiratory tract infections, pneumonia, Middle East Respiratory Syndrome, Severe Acute Respiratory Syndrome, Covid-19.

Hence, in a second aspect of the invention, the invention relates to a pharmaceutical composition comprising 4-aminoquinoline derivatives like Piperaquine, Chloroquine ((RS)-7-Chlor-4-(4-diethylamino-1-methylbutylamino)-chinolin) or Hydroxychloroquine (RS)-2-[{4-[(7-Chlor-4-chinolinyl)amino]pentyl}(ethyl)amino]ethanol) in combination with 3-N-formyl hydroxy amino propyl phosphonic acid derivatives or 3-N-acetyl hydroxy amino propyl phosphonic acid derivatives for use in the prophylaxis and treatment of infections caused by an RNA-virus, wherein the infection leads to a disease, in particular at least one disease selected from the group of respiratory diseases, lower respiratory tract infections, pneumonia, Middle East Respiratory Syndrome, Severe Acute Respiratory Syndrome, Covid-19.

The use of 3-N-formyl hydroxy amino propyl phosphonic acid derivatives and 3-N-acetyl hydroxy amino propyl phosphonic acid derivatives for prophylactic and therapeutic treatment of infectious processes, especially infections caused by viruses, fungi, parasites, is already known from DE19825585A1. However, DE19825585A1 is silent about RNA-viruses. An antibacterial activity has already been described in DE2733658.

Additionally, EP1071409B1 describes the use of 3-N-formyl hydroxy amino propyl phosphonic acid derivatives and 3-N-acetyl hydroxy amino propyl phosphonic acid derivatives for the treatment of infectious processes, especially infections caused by viruses. However, EP1071409B1 is silent about RNA-viruses.

In accordance with the invention the term "an RNA virus" shall mean a virus that has RNA (ribonucleic acid) as its genetic material and does not include DNA intermediates like a retrovirus. This nucleic acid is usually single-stranded RNA (ssRNA) but may be double-stranded RNA (dsRNA). Notable human diseases caused by RNA viruses include the common cold, influenza, SARS, COVID-19, Dengue fever, hepatitis C, hepatitis E, West Nile fever, Ebola virus disease, rabies, polio and measles. All said RNA-viruses may affect the ACE-2 (angiotensin-converting enzyme 2) receptor.

Fosmidomycin (FOS, 3-[Formyl(hydroxy)amino]propoylphosphonic acid, see below) exerts its anti-malarial activity by inhibition of 1-deoxy-D-xylulose 5-phosphate (DOXP) isomerase (Jomaa, H., J. Wiesner, S. Sanderbrand, B. Altincicek, C. Weidemeyer, M. Hintz, I. Turbachova, M. Eberl, J. Zeidler, H. K. Lichtenthaler, D. Soldati, and E. Beck. 1999. Inhibitors of the nonmevalonate pathway of isoprenoid biosynthesis as antimalarial drugs. Science 285:1573-6), an enzyme present in the Plasmodium apicoplast but absent from humans. Clinical trials established the drug's ability to rapidly clear parasites and reduce fever but recrudescence rates were high (Lell, B., R. Ruangweerayut, J. Wiesner, M. A. Missinou, A. Schindler, T. Baranek, M. Hintz, D. Hutchinson, H. Jomaa, and P. G. Kremsner. 2003. Fosmidomycin, a novel chemotherapeutic agent for malaria. Antimicrob Agents Chemother 47:735-8).

However, a virus has no 1-deoxy-D-xylulose 5-phosphate (DOXP) isomerase and no related pathway. But, surprisingly, 3-N-formyl hydroxy amino propyl phosphonic acid derivatives or 3-N-acetyl hydroxy amino propyl phosphonic acid derivatives in combination with 4-aminoquinoline derivatives are useful for the modulation of the ACE-2 (angiotensin-converting enzyme 2) receptor.

Even if all these compounds exhibit good results in the treatment of infections caused by RNA-viruses, there is an ongoing need to improve the efficacy of the drug. Hence, there is a large need to optimize and identify a drug combination having an improved anti-RNA-virus efficacy.

Therefore, the present invention relates to the object to enhance activity, stability and efficacy of a pharmaceutical preparation without increasing the side-effects of these active ingredients. Pharmaceutical preparations shall be made available providing a reduction of side-effects and improvement of efficacy. The object is as well to widen the range or therapeutic application of said pharmaceutical preparations and especially also to extend it to the treatment of problematic groups such as children and pregnant women. The anti-RNA-virus activity shall be increased to such a degree that these pharmaceutical preparations may be administered in lower doses and, thus, a reduction or elimination of side effects caused by these preparations is achieved. Moreover, the very preferred optimization shall be effected for the prophylaxis and treatment of infections caused by RNA-viruses and related diseases, in particular at least one disease selected from the group of respiratory diseases, lower respiratory tract infections, pneumonia, Middle East Respiratory Syndrome, Severe Acute Respiratory Syndrome, Covid-19.

Surprisingly, it has been found that 4-aminoquinoline derivatives selected from the group of Piperaquine, Chloroquine or Hydroxychloroquine in combination with one or more further pharmaceutical preparations / compounds resulting in a combination with 3-N-formyl hydroxy amino propyl phosphonic acid derivatives or 3-N-acetyl hydroxy amino propyl phosphonic acid derivatives are especially suited for the prophylaxis and treatment of infections caused by RNA-viruses and related diseases, in particular at least one disease selected from the group of respiratory diseases, lower respiratory tract infections, pneumonia, Middle East Respiratory Syndrome, Severe Acute Respiratory Syndrome, Covid-19.

According to the present invention 3-N-formyl-hydroxy amino propyl phosphonic acid derivatives and 3-N-acetyl hydroxy amino propyl phosphonic acid derivatives are deemed to be compounds of formula (II) 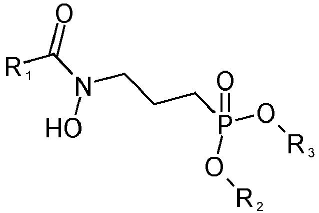
wherein R.sub.1 is selected from the group consisting of hydrogen and methyl, and
wherein R.sub.2 and R.sub.3 are independently selected from the group, consisting of hydrogen, substituted or unsubstituted alkyl, substituted or unsubstituted acyl, substituted or unsubstituted aryl, substituted or unsubstituted aralkyl, substituted or unsubstituted cycloalkyl, substituted or unsubstituted silyl, substituted or unsubstituted heterocyclic residue, or together form a substituted or unsubstituted C.sub.1-5-alkyl chain, the alkyl groups being saturated or comprising one or more double bonds or triple bonds.

However, in a preferred embodiment of the invention the formula I is fosmydomycin, (FOS) wherein R.sub.1 is hydrogen and R.sub.2 and R.sub.3 are hydrogen or a salt thereof, in particular 3-[Formyl(hydroxy)amino]propoylphosphonic acid (CAS No. 66508-53-0), particularly a sodium salt thereof.

The disclosed preparations / pharmaceutical compositions in accordance with the invention are also deemed to be the respective salts (e.g. sodium salts or the like). In a preferred embodiment the salt is a piperaquine tetraphosphate with four waters of crystallization.

Hence, all mentioned preparations / pharmaceutical compositions may comprise, if appropriate, a salt thereof and, if required, further adjuvants.

Special features of the above definitions and suitable examples thereof are stated below:
"Acyl" is a substituent which originates from an acid, such as from an organic carboxylic acid, carbonic acid, carbamic acid or the thioacid or imidic acid corresponding to the individual above-stated acids, or from an organic sulfonic acid, wherein these acids may in each case comprise aliphatic, aromatic and/or heterocyclic groups in the molecule, as well as carbamoyl or carbamimidoyl.

Suitable examples of these acyl groups are stated below. Aliphatic acyl groups are deemed to comprise acyl residues originating from an aliphatic acid, such groups including the following:
alkanoyl (for example formyl, acetyl, propionyl, butyryl, isobutyryl, valeryl, isovaleryl, pivaloyl etc.);
alkenoyl (for example acryloyl, methacryloyl, crotonoyl etc.);
alkylthioalkanoyl (for example methylthioacetyl, echylthioacetyl etc.);
alkanesulfonyl (for example mesyl, echanesulfonyl, propanesulfonyl etc.);
alkoxycarbonyl (for example methoxycarbonyl, ethoxycarbonyl, propoxycarbonyl, isopropoxycarbonyl, butoxycarbonyl, isobutoxycarbonyl etc.);
alkylcarbamoyl (for example methylcarbamoyl etc.);
(N-alkyl)thiocarbamoyl (for example (N-methyl)thiocarbamoyl etc.);
alkylcarbamimidoyl (for example methylcarbamimidoyl etc.);
oxalo;
alkoxalyl (for example methoxalyl, ethoxalyl, propoxalyl etc.).

In the above examples of aliphatic acyl groups, the aliphatic hydrocarbon moiety, in particular the alkyl group or alkane residue, may optionally comprise one or more suitable substituents, such as amino, halogen (for example fluorine, chlorine, bromine etc.), hydroxy, hydroxyimino, carboxy, alkoxy (for example methoxy, ethoxy, propoxy etc.), alkoxycarbonyl, acylamino (for example benzyloxycarbonylamino etc.), acyloxy (for example acetoxy, benzyloxy etc.) and the like; preferred aliphatic acyl residues having such substituents which may be mentioned are alkanoyls substituted, for example, with amino, carboxy, amino and carboxy, halogen, acylamino or the like.

Aromatic acyl residues are deemed to comprise those acyl residue which originate from an acid with a substituted or unsubstituted aryl group, wherein the aryl group may comprise phenyl., toluyl, xylyl, naphthyl and the like; suitable examples are stated below:
aroyl (for example benzoyl, toluoyl, xyloyl, naphthoyl, phthaloyl etc.);
aralkanoyl (for example phenylacetyl etc.);
aralkenoyl (for example cinnamoyl etc.);
aryloxyalkanoyl (for example phenoxyacetyl etc.);
arylthioalkanoyl (for example phenylthioacetyl etc.);
arylaminoalkanoyl (for example N-phenylglycyl etc.);
arenesulfonyl (for example benzenesulfonyl, tosyl or toluenesulfonyl, naphthalenesulfonyl etc.);
aryloxycarbonyl (for example phenoxycarbonyl, naphthyloxycarbonyl etc.);
aralkoxycarbonyl (for example benzyloxycarbonyl etc.);
arylcarbamoyl (for example phenylcarbamoyl, naphthylcarbamoyl etc.);
arylglyoxyloyl (for example phenylglyoxyloyl etc.).

In the above examples of acyl residues, the aromatic hydrocarbon moiety (in particular the aryl residue) and/or the aliphatic hydrocarbon moiety (in particular the alkane residue) may optionally comprise one or more suitable substituents, such as those which have already been stated as suitable substituents for the alkyl group or the alkane residue. Aromatic acyl residues having particular substituents which may in particular be mentioned and constitute examples of preferred aromatic acyl residues are aroyl substituted with halogen and hydroxy or with halogen and acyloxy, and aralkanoyl substituted with hydroxy, hydroxyimino, dihaloalkanoyloxyimino, together with
arylthiocarbamoyl (for example phenylthiocarbamoyl etc.);
arylcarbamimidoyl (for example phenylcarbamimidoyl etc.).

A heterocyclic acyl residue is taken to mean an acyl residue which originates from an acid with a heterocyclic group; these include:
heterocyclic carbonyl, wherein the heterocyclic residue is an aromatic or aliphatic 5-to 6-membered heterocycle with at least one heteroatom from the group comprising nitrogen, oxygen and sulphur (for example thiophenyl, furoyl, pyrrolocarbonyl, nicotinoyl etc.);
alkanoyl heterocycle, wherein the heterocyclic residue is 5- to 6-membered and comprises at least one heteroatom from the group comprising nitrogen, oxygen and sulphur (for example thiophenylacetyl, furylacetyl, imidazolylpropionyl, tetrazolylacetyl, 2-(2-amino-4-thiazolyl)-2-methoxyiminoacetyl etc.) and the like.

In the above examples of heterocyclic acyl residues, the heterocycle and/or the aliphatic hydrocarbon moiety may optionally comprise one or more suitable substituents, such as chose as have been stated to be suitable for alkyl and alkane groups.

"Alkyl groups" are straight- or branched-chain alkyl residues having 1 to 24 carbon atoms, such as methyl, ethyl, propyl, isopropyl, butyl, isobutyl, tert.-butyl, pentyl, hexyl and the like. They may be e.g. substituted with hydroxy, halogen or oxy groups.

"Cycloalkyl" preferably represents a optionally substituted C.sub.3-8-cycloalkyl; a. o. alkoxy (e.g. methoxy, ethoxy, etc.), halogen (e.g. fluorine, chlorine, bromine etc.), nitro and the like are suited to be possible substituents.

"Aryl" is an aromatic hydrocarbon residue, such as phenyl, naphthyl etc., which may optionally comprise one or more suitable substituents such as alkyl, alkoxy (for example methoxy, ethoxy etc.), trifluoromethylene, halogen (for example fluorine, chlorine, bromine etc.), nitro and the like.

"Aralkyl" includes mono-, di-, triphenylalkyl such as benzyl, phenethyl, benzhydryl, trityl and the like wherein the aromatic moiety may optionally comprise one or more suitable substituents such as alkoxy (for example methoxy, ethoxy etc.), halogen (for example fluorine, chlorine, bromine etc.), nitro and the like.

In the above ester the alkane and/or arene moiety may optionally comprise at least one suitable substituent, such as halogen, alkoxy, hydroxy, nitro and the like.

The use of combination therapy with the help of pharmaceutical preparations of the present invention has the advantage of a synergistic increase of antiviral activity of the single substances. Hence, in combining the single compounds, there is a possibility of reducing the doses and, thus, the toxicity of the single compounds at the same time preserving antiviral activity.

With the use of said combination therapy it is possible to administer the active agents in a so-called fixed combination, i.e. in a single pharmaceutical formulation containing the active agents or to choose a so-called free combination, administering the active agents in form of separate pharmaceutical formulations at the same time or one after the other (e.g. in form of a blister).

If the active agents are solid materials, the active agents may be administered by conventional methods for solid drug preparations mixing e.g. all active agents and pelletizing them for example into pellets together with conventional excipients or auxiliary materials. However, it is also possible to provide the active agents separately in one package unit ready for sale wherein the package unit contains all active agents in separate pharmaceutical formulations.

The pharmaceutical preparations may be administered in liquid or solid form for enteral or parenteral application. In this connection all conventional forms of application are possible, for example pellets, capsules, dragees, sirups, solutions, suspensions. Preferably, water is used as an injection medium containing added substances common in injection solutions such as stabilizers, dissolving intermediaries and buffers. If desired, preparations suited for oral application may contain flavorings or sweeteners.

Moreover, it is found that severe forms of Covid-19 are marked by occurrence of neutrophil precursors, as evidence of emergency myelopoiesis, dysfunctional mature neutrophils, and *HL A-DR^{lo}* monocytes leading to increased neutrophil counts and dysregulated immune responses (Jonas Schulte-Schrepping, Nico Reusch, Daniela Paclik, Kevin Baßler, Stephan Schlickeiser, Bowen Zhang, Benjamin Kramer, Tobias Krammer, Sophia Brumhard, Lorenzo Bonaguro, Elena De Domenico, Daniel Wendisch, Martin Grasshoff, Theodore S. Kapellos, Michael Beckstette, Tal Pecht, Adem Saglam, Oliver Dietrich, Henrik E. Mei, Axel R. Schulz, Claudia Conrad, Désirée Kunkel, Ehsan Vafadarnejad, Cheng-Jian Xu, Arik Horne, Miriam Herbert, Anna Drews, Charlotte Thibeault, Moritz Pfeiffer, Stefan Hippenstiel, Andreas Hocke, Holger Muller-Redetzky, Katrin-Moira Heim, Felix Machleidt, Alexander Uhrig, Laure Bosquillon de Jarcy, Linda Jürgens, Miriam Stegemann, Christoph R. Glosenkamp, Hans-Dieter Volk, Christine Goffinet, Markus Landthaler, Emanuel Wyler, Philipp Georg, Maria Schneider, Chantip Dang-Heine, Nick Neuwinger, Kai Kappert, Rudolf Tauber, Victor Corman, Jan Raabe, Kim Melanie Kaiser, Michael To Vinh, Gereon Rieke, Christian Meisel, Thomas Ulas, Matthias Becker, Robert Geffers, Martin Witzenrath, Christian Drosten, Norbert Suttorp, Christof von Kalle, Florian Kurth, Kristian Handler, Joachim L. Schultze, Anna C. Aschenbrenner, Yang Li, Jacob Nattermann, Birgit Sawitzki, Antoine-Emmanuel Saliba, Leif Erik Sander, Deutsche COVID-19 OMICS Initiative (DeCOI). Severe COVID-19 is marked by a dysregulated myeloid cell compartment. Cell (2020), doi: https://doi.org/10.1016/j.cell. 2020.08.001).

For that reason, the inventors have found to add a further compound selected from the group of immunosuppressive drugs to re-balance the dysfunctional immune response of an infected patient.

Hence, the present invention refers to pharmaceutical preparations / compositions comprising 4-aminoquinoline derivatives, in particular Piperaquine, Chloroquine, or Hydroxychloroquine as active ingredients, preferably in a further embodiment in combination with 3-N-formyl hydroxy amino propyl phosphonic acid derivatives or 3-N-acetyl hydroxy amino propyl phosphonic acid derivatives as active ingredients or optionally, in combination with an immunosuppressant for use in the prophylaxis and treatment of infections caused by RNA-viruses.

In a further preferred embodiment of the invention the immunosuppressive drugs are selected from the group of IMDH inhibitors, calcineurin inhibitors, corticosteroids, mTOR inhibitors, or Cyclophosphamide, or a combination thereof.

In a further preferred embodiment of the invention, IMDH inhibitors are selected from the group of Methotrexate, Mycophenolate, Azathiaprine and Leflunomide.

In a further preferred embodiment of the invention, calcineurin inhibitors are selected from the group of Ciclosporin and Tacrolimus.

In a further preferred embodiment of the invention, corticosteroids are selected from the group of Prednisone, Budesonide and Prednisolone.

In a further preferred embodiment of the invention, mTOR inhibitors are selected from the group of the Janus kinase inhibitor, Tofacitinib, Sirolimus and Everolimus.

In the following, the present invention is described in more detail by way of examples. However, these examples are not intended to limit the scope of protection of the present invention in any way.

### EXAMPLES

### Case history.

A patient suffering from malaria was found to have a coincidental Covid-19 infection and was treated successfully with piperaquine and fosmidomycin in doses of 16 mg/kg and 30 mg/kg respectively for three days.

Presenting with high fever, dry cough, fatigue and myalgia consistent with Covid-19, a routine blood smear was positive for Plasmodium falciparum infection. The simultaneous coinfection with Covid-19 was confirmed by a PCR test according to Drosten et al (Corman et al Detection of 2019 novel coronavirus (2019-nCoV) by real-time RT-PCR, Euro Surveill. 2020 Jan 23; 25(3): 2000045.doi: 10.2807/1560-7917.ES.2020.25.3.2000045),

Treatment was initiated with the previously successfully applied combination of piperaquine and fosmidomycin against P. falciparum malaria and resulted in a satisfactory parasitological outcome and clinical response with respect to fever, shortness of breath and fatigue. The medication was well tolerated and there were no safety concerns.

It was concluded that the combination of piperaquine and fosmidomycin surprisingly possesses dual activity against Plasmodium falciparum and Covid-19.

## Claims

1. Pharmaceutical composition comprising a 4-aminoquinoline derivative, namely Piperaquine for use in the prophylaxis and treatment of infections caused by an RNA-virus and, if appropriate, a salt thereof and, if required, further adjuvants.

2. Pharmaceutical composition comprising a 4-aminoquinoline derivative, namely Piperaquine for use in the prophylaxis and treatment of infections caused by an RNA-virus according to claim 1, wherein the RNA-virus is selected from the group of coronavirus (CoV), Middle East Respiratory Syndrome (MERS)-CoV or Severe Acute Respiratory Syndrome (SARS)-CoV, Severe Acute Respiratory Syndrome (SARS)-CoV-2.

3. Pharmaceutical composition comprising a 4-aminoquinoline derivative, namely Piperaquine for use in the prophylaxis and treatment of infections caused by an RNA-virus according to claim 1 or claim 2, wherein the infections are related to at least one disease selected from the group of respiratory diseases, lower respiratory tract infections, pneumonia, Middle East Respiratory Syndrome, Severe Acute Respiratory Syndrome, Covid-19.

4. Pharmaceutical composition comprising a 4-aminoquinoline derivative selected from the group of Piperaquine, Chloroquine or Hydroxychloroquine in combination with 3-N-formyl hydroxy amino propyl phosphonic acid derivatives or 3-N-acetyl hydroxy amino propyl phosphonic acid derivatives
of the formula (II) 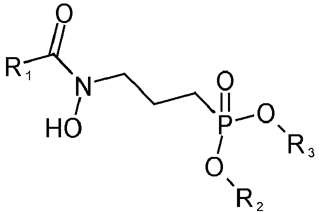 wherein R.sub.1 is selected from the group consisting of hydrogen and methyl, and
wherein R.sub.2 and R.sub.3 are independently selected from the group, consisting of hydrogen, substituted or unsubstituted alkyl, substituted or unsubstituted acyl, substituted or unsubstituted aryl, substituted or unsubstituted aralkyl, substituted or unsubstituted cycloalkyl, substituted or unsubstituted silyl, substituted or unsubstituted heterocyclic residue, or together form a substituted or unsubstituted C.sub.1-5-alkyl chain, the alkyl groups being saturated or comprising one or more double bonds or triple bonds,
for use in the prophylaxis and treatment of infections caused by an RNA-virus, in particular an RNA-virus according to claim 2 and / or in particular a related disease according to claim 3
and, if appropriate, a salt thereof and, if required, further adjuvants.

5. Pharmaceutical composition in accordance with claim 4, wherein the formula II represents fosmidomycin or a salt thereof.

6. Pharmaceutical composition in accordance with one of the preceding claims, wherein at least one further compound is selected from at least one immunosuppressive drug.

7. Pharmaceutical composition in accordance with claim 6, wherein the at least one immunosuppressive drug is selected from the group of IMDH inhibitors, calcineurin inhibitors, corticosteroids, mTOR inhibitors, or Cyclophosphamide, or a combination thereof.

8. Pharmaceutical composition in accordance with claim 7, wherein the at least one immunosuppressive drug is selected from the group of Methotrexate, Mycophenolate, Azathiaprine, Leflunomide, Ciclosporin, Tacrolimus, Prednisone, Budesonide and Prednisolone, Janus kinase inhibitor, Tofacitinib, Sirolimus and Everolimus.
